# EUROPÄISCHE PATENTSCHRIFT

(11) **EP 3 247 289 B1**
(45) Veröffentlichungstag und Bekanntmachung des Hinweises auf die Patenterteilung: **12.06.2024**
(21) Anmeldenummer: 15790879.9
(22) Anmeldetag: 29.10.2015
(51) Int. Cl.: A61B 17/29, A61B 90/00, A61B 17/28, A61B 17/16

(54) **HANDGRIFFVORRICHTUNG**
HANDLE DEVICE
SYSTÈME DE POIGNÉE

(30) Priorität: 22.01.2015 DE 102015100945
(43) Veröffentlichungstag der Anmeldung: 29.11.2017
(73) Patentinhaber: STERIS Deutschland GmbH, 50933 Köln (DE)
(72) Erfinder: DANNORITZER, Axel, 78532 Tuttlingen (DE); RAUCH, Stefan, 78532 Tuttlingen (DE); FUCHS, Harald, 78532 Tuttlingen (DE); KLAIBER, Gerlinde, 78595 Hausen (DE); DANNORITZER, Julian, 78573 Wurmlingen (DE)
(74) Vertreter: Daub, Thomas
(86) Internationale Anmeldenummer: PCT/EP2015/075121
(87) Internationale Veröffentlichungsnummer: WO 2016/116179

(56) Entgegenhaltungen:
- WO-A1-01/35838
- DE-U1- 29 619 246
- DE-U1-202011 052 256
- US-A- 5 584 844
- US-A- 6 077 290
- US-A1- 2009 209 998
- US-A1- 2014 236 204

## Beschreibung

### Stand der Technik

Die Erfindung betrifft eine Handgriffvorrichtung, insbesondere eine Chirurgiewerkzeug-Handgriffvorrichtung.

In der DE 10 2008 058 207 A1 ist bereits eine Handgriffvorrichtung, insbesondere eine Chirurgiewerkzeug-Handgriffvorrichtung, mit einem ersten und zumindest einem zweiten Griffelement und einem Scherengelenk, mittels dessen die zwei Griffelemente schwenkbar verbunden sind, sowie mit einer Kopplungseinheit, die dazu vorgesehen ist, die beiden Griffelemente zusammenzuhalten, wobei die Kopplungseinheit wenigstens ein Kopplungselement aufweist, das dazu vorgesehen ist, abhängig von einer Relativposition der Griffelemente eine Formschlussverbindung derselben herzustellen, vorgeschlagen worden.

DE 20 2011 052256 U1 offenbart ein medizinisches Instrument gemäß dem Oberbegriff des Anspruchs 1.

Die Aufgabe der Erfindung besteht insbesondere darin, eine gattungsgemäße Vorrichtung mit verbesserten Eigenschaften hinsichtlich einer einfachen Konstruktion bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Handgriffvorrichtung gemäß Anspruch 1, insbesondere einer Chirurgiewerkzeug-Handgriffvorrichtung, mit einem ersten Griffelement und einem zweiten Griffelement und einem Scherengelenk, mittels dessen die zwei Griffelemente schwenkbar und separierbar verbunden sind, sowie mit einer Kopplungseinheit , die dazu vorgesehen ist, die beiden Griffelemente entgegen einer Separierrichtung zusammenzuhalten, und die wenigstens ein Kopplungselement aufweist, das dazu vorgesehen ist, abhängig von einer Relativposition der Griffelemente eine Formschlussverbindung derselben entgegen der Separierrichtung herzustellen, wobei das das wenigstens eine Kopplungselement als Zentrierelement zur Zentrierung der Formschlussverbindung ausgebildet ist.

Es wird vorgeschlagen, dass die Handgriffvorrichtung zumindest ein korrespondierendes Kopplungselement aufweist, welches eine zumindest teilweise kreisförmige Ausnehmung zur Aufnahme des Kopplungselements begrenzt, wobei das Kopplungselement und das korrespondierende Kopplungselement zur Herstellung der Formschlussverbindung mittels einer Verschiebebewegung in einer Ebene zumindest im Wesentlichen parallel zu einer Drehebene des Scherengelenks vorgesehen sind. Dadurch kann eine Anzahl von Bauteilen der Handgriffvorrichtung reduziert werden. Ferner kann eine einfache Führung einer Drehbewegung der Griffelemente bei einer Betätigung der Handgriffvorrichtung in einem Scherengriff erreicht werden. Des Weiteren kann eine Handgriffvorrichtung erreicht werden, deren einzelne Bauteile für eine getrennte Reinigung leicht separiert werden können. Ferner kann ein konstruktiv besonders einfach ausgeführtes korrespondierendes Kopplungselement erreicht werden. Zudem kann eine besonders einfache Bewegung zur Herstellung der Formschlussverbindung erreicht werden
Unter einem "Scherengelenk" soll in diesem Zusammenhang insbesondere eine mechanische Verbindung der Griffelemente verstanden werden, die eine relative Drehbewegung der beiden Griffelemente um eine gemeinsame Drehachse, die senkrecht zu der von den beiden Griffelementen aufgespannten Ebene und innerhalb ihrer Verbindung liegt, ermöglicht. Unter "vorgesehen" soll insbesondere speziell programmiert, ausgelegt und/oder ausgestattet verstanden werden. Darunter, dass ein Objekt zu einer bestimmten Funktion vorgesehen ist, soll in diesem Zusammenhang verstanden werden, dass das Objekt diese bestimmte Funktion in zumindest einem Anwendungs- und/oder Betriebszustand erfüllt und/oder ausführt. Darunter, dass die zwei Griffelemente mittels des Scherengelenks "separierbar verbunden" sind, soll in diesem Zusammenhang verstanden werden, dass die zwei Griffelemente durch Umkehrung einer Montagebewegung voneinander getrennt werden können. Unter einer "Separierrichtung" soll in diesem Zusammenhang eine Richtung verstanden werden, entlang der eine Trennung der zwei Griffelemente vorgesehen ist. Darunter, dass "die Kopplungseinheit dazu vorgesehen ist, die beiden Griffelemente entgegen einer Separierrichtung zusammenzuhalten", soll in diesem Zusammenhang verstanden werden, dass die Kopplungseinheit die beiden Griffelemente derart zusammenhält, dass diese lediglich in einer bestimmten Winkelstellung zueinander entlang der Separierrichtung voneinander separiert werden können und Bewegungen der Griffelemente im montierten Zustand entgegen der Separierrichtung von der Kopplungseinheit blockiert werden. Insbesondere ist das Kopplungselement an einem der Griffelemente fest montiert, insbesondere eingesteckt. Grundsätzlich kann das Kopplungselement einstückig an dem einen Griffelement angeordnet sein, beispielsweise durch Anschweißen, oder das Kopplungselement kann als Ausformung eines der Griffelemente ausgeführt sein.

Unter einer "zumindest teilweise kreisförmigen Ausnehmung" soll in diesem Zusammenhang eine Ausnehmung verstanden werden, die einen Teilbereich aufweist, der durch eine kreisförmige Kontur begrenzt wird, welche entlang maximal einem Drittel, bevorzugt entlang maximal einem Sechstel eines Kreisumfangs geöffnet ist, wobei sich weitere Teilbereiche der Ausnehmung an einer Öffnung der kreisförmigen Kontur an den Teilbereich, der durch die kreisförmige Kontur begrenzt wird, anschließen. Darunter, dass die Verschiebebewegung "in einer Ebene zumindest im Wesentlichen parallel zu einer Drehebene des Scherengelenks" verläuft, soll in diesem Zusammenhang verstanden werden, dass eine Richtung, in der die Verschiebebewegung verläuft, mit einer Ebene parallel zu der Drehebene des Scherengelenks einen Winkel von maximal fünf Grad, bevorzugt von maximal drei Grad und besonders bevorzugt von maximal einem Grad, einschließt. Insbesondere kann die Verschiebebewegung vollständig innerhalb der Ebene parallel zu der Drehebene des Scherengelenks verlaufen.

Gemäß der Erfindung wird vorgeschlagen, dass das wenigstens eine Kopplungselement zumindest einen Bereich aufweist, der in einer ersten Querrichtung eine erste Erstreckung und in einer zweiten Querrichtung eine zweite Erstreckung aufweist, die größer ist als die erste Erstreckung. Dadurch kann auf eine konstruktiv einfache Weise eine Separierrichtung angezeigt werden. Ferner kann auf eine konstruktiv einfache Weise eine Blockade einer Abziehbewegung in einer Richtung, die zumindest teilweise senkrecht zu einer vorgesehenen Separierrichtung verläuft, erreicht werden. Unter einer "Querrichtung" soll in diesem Zusammenhang eine Richtung verstanden werden, die in einem montierten Zustand des Kopplungselements in einer Ebene senkrecht zu einer Rotationsachse des Scherengelenks verläuft. Insbesondere verlaufen die Querrichtungen senkrecht zu einer Montagerichtung des Kopplungselements an einem der Griffelemente. Unter einer "ersten Querrichtung" und einer "zweiten Querrichtung" sollen in diesem Zusammenhang Querrichtungen verstanden werden, die senkrecht zueinander verlaufen. Insbesondere werden die zwei Griffelemente mit einer Bewegung, die senkrecht zu der ersten Querrichtung verläuft, montiert.

Des Weiteren wird vorgeschlagen, dass das wenigstens eine Kopplungselement an Enden des Bereichs abgerundet ausgeführt ist. Dadurch kann eine einfache Montierbewegung erreicht und ein Verkanten des Kopplungselements und eines der Griffelemente während der Montage vermieden werden.

Weiterhin wird vorgeschlagen, dass das korrespondierende Kopplungselement einen Führungskanal zur Führung des Kopplungselements in die zumindest teilweise kreisförmige Ausnehmung begrenzt. Dadurch kann auf konstruktiv einfache Weise ein Führungselement zur Führung des Kopplungselements bereitgestellt werden. Ferner kann eine intuitive Führung der Montagebewegung bei einem Zusammenbau der Handgriffvorrichtung erreicht werden.

Des Weiteren wird vorgeschlagen, dass das korrespondierende Kopplungselement ein weiteres Zentrierelement ausbildet. Dadurch kann eine Anzahl von Bauteilen der Handgriffvorrichtung reduziert werden. Ferner kann eine besonders einfache Zentrierung des Scherengelenks erreicht werden.

Des Weiteren wird vorgeschlagen, dass das Kopplungselement und das korrespondierende Kopplungselement zumindest eine Arretierungseinheit aufweisen, die dazu vorgesehen ist, die Griffelemente in zumindest einer Arretierungsstellung zu arretieren. Dadurch können die Griffelemente in einer Stellung gesichert werden, die einen korrekten Zusammenbau der Griffelemente in einer Stellung, in der das Werkzeug korrekt montiert werden kann, ermöglicht.

Weiterhin wird vorgeschlagen, dass die Handgriffvorrichtung eine Werkzeugeinheit und eine Sicherungseinheit aufweist, die einen Verschlusshebel umfasst, der eine Schwenkachse zur Schwenkung zwischen einer Freigabestellung und einer Befestigungsstellung für die Werkzeugeinheit aufweist, und die zur reversiblen Halterung der Werkzeugeinheit an zumindest einem der Griffelemente vorgesehen ist. Dadurch kann eine Demontage der Werkzeugeinheit, beispielsweise für eine Reinigung, getrennt von den Griffelementen erreicht werden. Ferner kann eine werkzeuglose Demontage der Werkzeugeinheit erreicht werden. Unter einer "Werkzeugeinheit" soll in diesem Zusammenhang eine Einheit verstanden werden, die zur Halterung in zumindest einem der Griffelemente vorgesehen ist und die zumindest zur Halterung eines Werkzeugs vorgesehen ist oder einstückig mit dem Werkzeug ausgebildet ist. Insbesondere umfasst die Werkzeugeinheit Bauteile zur Betätigung des Werkzeugs. Unter einem "Verschlusshebel" soll in diesem Zusammenhang eine Einheit verstanden werden, die schwenkbar gelagert ist und die zumindest die Werkzeugeinheit und gegebenenfalls weitere Bauteile der Handgriffvorrichtung zumindest in einer festen Position lagert. Insbesondere werden durch Schwenkung des Verschlusshebels die Werkzeugeinheit und gegebenenfalls weitere Bauteile der Handgriffvorrichtung freigegeben, sodass sie zumindest gereinigt und bevorzugt demontiert werden können. Insbesondere wird durch die Schwenkung zwischen der Befestigungsstellung und der Freigabestellung eine Bewegung ausgelöst, die zumindest teilweise, bevorzugt vollständig, entlang einer Erstreckungsrichtung der Schwenkachse erfolgt.

Des Weiteren wird vorgeschlagen, dass die Sicherungseinheit ein Kurvengetriebe mit zwei aufeinander gleitenden Schrägflächen aufweist, die bei Schwenkung des Verschlusshebels in die Freigabestellung zur Freigabe der Werkzeugeinheit aneinander abgleiten. Dadurch kann eine einfache Konstruktion der Sicherheitseinheit erreicht werden.

Weiterhin wird vorgeschlagen, dass die Sicherungseinheit ein Gewinde und eine Gewindeschraube, die bei Schwenkung des Verschlusshebels aus dem Gewinde ausgeschraubt wird und die Werkzeugeinheit freigibt, aufweist. Dadurch kann eine konstruktiv besonders einfach ausgeführte Sicherungseinheit erreicht werden.

Ferner wird vorgeschlagen, dass die Werkzeugeinheit einen Schaft zur Aufnahme eines Werkzeugs und einen Anschlag umfasst, an dem der Schaft gelagert ist, und der Verschlusshebel zur direkten Befestigung des Anschlags an einem der Griffelemente in der Befestigungsstellung und für eine Freigabe des Anschlags in der Freigabestellung vorgesehen ist. Dadurch kann eine konstruktiv besonders einfach ausgeführte Sicherungseinheit erreicht werden.

Des Weiteren wird vorgeschlagen, dass die Werkzeugeinheit zumindest einen Aufsatz umfasst, der mit dem ersten Griffelement ein Werkzeug ausbildet und der in der Befestigungsstellung von dem Verschlusshebel an dem ersten Griffelement befestigt wird.

Dadurch kann eine konstruktiv besonders einfach ausgeführte Sicherungseinheit erreicht werden.

Weiterhin wird vorgeschlagen, dass die Sicherungseinheit einen weiteren Verschlusshebei umfasst. Dadurch kann eine Sicherungseinheit mit einer erhöhten Sicherheit erreicht werden.

Ferner wird ein System mit einer Handgriffvorrichtung nach einem der vorhergehenden Ansprüche und einem Werkzeug, insbesondere einem Chirurgiewerkzeug, vorgeschlagen.

### Zeichnungen

Weitere Vorteile ergeben sich aus der folgenden Zeichnungsbeschreibung. In den Zeichnungen sind vier Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Handgriffvorrichtung gemäß einem ersten Ausführungsbeispiel der Erfindung,
- Fig. 2: eine Detaildarstellung eines Ausschnitts der Handgriffvorrichtung des ersten Ausführungsbeispiels mit einer Sicherungseinheit, die einen Verschlusshebel umfasst,
- Fig. 3: eine Detaildarstellung eines Kopplungselements der Handgriffvorrichtung,
- Fig. 4: eine Detaildarstellung eines ersten Griffelements und eines zweiten Griffelements,
- Fig. 5: eine Detaildarstellung einer Werkzeugeinheit der Handgriffvorrichtung, mit einem Schieber, mit einem mit dem Schieber fest verbundenen und beweglichen Halteelement und mit einem Schaft, in dem der Schieber gelagert ist und der eine Nut zur Führung des Halteelements aufweist,
- Fig. 6: eine Handgriffvorrichtung gemäß einem zweiten Ausführungsbeispiel der Erfindung,
- Fig. 7: eine Detaildarstellung eines Ausschnitts der Handgriffvorrichtung des zweiten Ausführungsbeispiels mit einer Sicherungseinheit, die einen Verschlusshebel umfasst, und
- Fig. 8: eine Detaildarstellung der Bereiche eines ersten Griffelements und eines zweiten Griffelements mit einem Kopplungselement und einem korrespondierenden Kopplungselement,
- Fig. 9: eine Detaildarstellung eines dritten Ausführungsbeispiels mit zwei Verschlusshebeln,
- Fig. 10: eine Schnittdarstellung der zwei Verschlusshebel des dritten Ausführungsbeispiels,
- Fig. 11: eine schematische Darstellung eines vierten Ausführungsbeispiels mit zwei Verschlusshebeln und
- Fig. 12: eine Schnittdarstellung der zwei Verschlusshebel des vierten Ausführungsbeispiels.

### Beschreibung der Ausführungsbeispiele

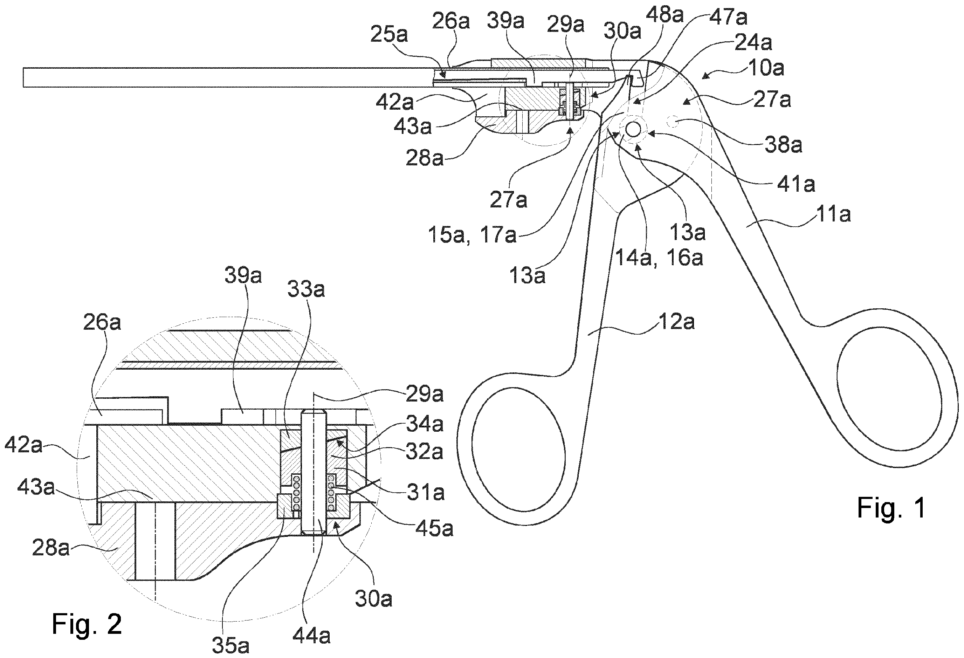

Die Figuren 1 und 2 zeigen eine als Chirurgiewerkzeug-Handgriffvorrichtung ausgeführte Handgriffvorrichtung 10a, mit einem ersten Griffelement 11a und einem zweiten Griffelement 12a und einem Scherengelenk, mittels dessen die zwei Griffelemente 11a, 12a schwenkbar und separierbar verbunden sind, sowie mit einer Kopplungseinheit 13a, die dazu vorgesehen ist, die beiden Griffelemente 11a, 12a entgegen einer Separierrichtung zusammenzuhalten, und die ein Kopplungselement 14a aufweist, das dazu vorgesehen ist, abhängig von einer Relativposition der Griffelemente 11a, 12a eine Formschlussverbindung derselben entgegen der Separierrichtung herzustellen. Das erste Griffelement 11a ist als Hauptteil der Handgriffvorrichtung 10a ausgeführt und ist zur Aufnahme eines Werkzeugs vorgesehen. Das zweite Griffelement 12a ist dazu vorgesehen, auf das erste Griffelement 11a an der Kopplungseinheit 13a aufgesteckt zu werden. Das erste Griffelement 11a und das zweite Griffelement 12a sind aus Stahl hergestellt.

Das Kopplungselement 14a ist als Zentrierelement 16a zur Zentrierung der Formschlussverbindung ausgebildet. Bei einer Betätigung der Handgriffvorrichtung 10a wird das zweite Griffelement 12a relativ zu dem ersten Griffelement 11a rotiert, wobei eine Rotation um eine Drehachse des Scherengelenks erfolgt, die zentral durch das als Zentrierelement 16a ausgeführte Kopplungselement 14a verläuft. Das zweite Griffelement 12a wird durch das als Zentrierelement 16a ausgeführte Kopplungselement 14a in seiner Bewegung geführt, wobei das als Zentrierelement 16a ausgeführte Kopplungselement 14a das zweite Griffelement 12a dabei kontaktiert und eine Bewegung des zweiten Griffelements 12a in eine Richtung senkrecht zu der Drehachse des Scherengelenks mit Ausnahme einer Bewegung in Separierrichtung blockiert.

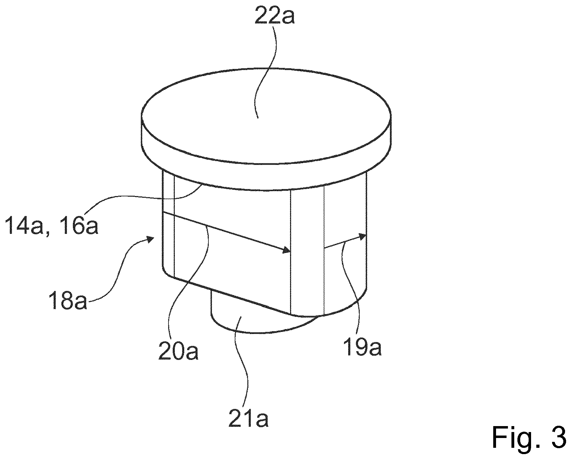

Das Kopplungselement 14a weist einen Bereich 18a auf, der in einer ersten Querrichtung 19a eine erste Erstreckung und in einer zweiten Querrichtung 20a eine zweite Erstreckung aufweist, die größer ist als die erste Erstreckung (Fig. 3). Die zweite Erstreckung beträgt das 1,9-fache der ersten Erstreckung. Das Kopplungselement 14a ist an Enden des Bereichs 18a in einer Ebene, in der die Querrichtungen 19a, 20a verlaufen, abgerundet ausgeführt. Eine Abrundung der Enden des Bereichs 18a ist als zweistufiger Abschliff ausgeführt. In alternativen Ausführung kann das Kopplungselement 14a an Enden des Bereichs 18a vollständig rundgeschliffen oder in mehreren Stufen abgerundet ausgeführt sein.

In einer Richtung senkrecht zu der ersten Querrichtung 19a und der zweiten Querrichtung 20a weist das Kopplungselement 14a an einem Endstück eine den Bereich 18a überstehende Krempe 22a mit einem kreisförmigen Querschnitt auf. Die Krempe 22a blockiert eine Abhebebewegung des ersten Griffelements 11a und des zweiten Griffelements 12a voneinander entlang der Drehachse des Scherengelenks. An einem von der Krempe 22a abgewandten Endstück des Kopplungselements 14a weist das Kopplungselement 14a einen Einsteckpin 21a auf, der zum Einstecken in eine korrespondierende Bohrung des ersten Griffelements 11a vorgesehen ist. Der Einsteckpin 21a wird nach Einstecken in die korrespondierende Bohrung an das erste Griffelement 11a geschweißt. Die Bohrung ist in einem Zentrum einer rechteckigen Ausfräsung 41a des ersten Griffelements 11a ausgeführt. Die rechteckige Ausfräsung 41a entspricht in äußeren Abmessungen einem Querschnitt des Bereichs 18a des Kopplungselements 14a mit den Querrichtungen 19a, 20a. Das Kopplungselement 14a wird mit dem Bereich 18a und dem Einsteckpin 21a in die rechteckige Ausfräsung 41a und die Bohrung eingesetzt und ist im eingesetzten Zustand teilweise in die rechteckige Ausfräsung 41a versenkt, übersteht jedoch eine Oberfläche des ersten Griffelements 11a in einer Umgebung um die rechteckige Ausfräsung 41a.

Eine Position und eine Ausrichtung des Kopplungselements 14a im montierten Zustand an dem ersten Griffelement 11a werden durch die rechteckige Ausfräsung 41a und die Bohrung eindeutig festgelegt.

Die Handgriffvorrichtung 10a umfasst ein korrespondierendes Kopplungselement 15a, welches eine teilweise kreisförmige Ausnehmung 23a zur Aufnahme des Kopplungselements 14a begrenzt. Das korrespondierende Kopplungselement 15a ist als Teilbereich des zweiten Griffelements 12a ausgeführt, der die teilweise kreisförmige Ausnehmung 23a aufweist. Die teilweise kreisförmige Ausnehmung 23a weist einen Teilbereich auf, der durch eine kreisförmige Kontur begrenzt wird, welche entlang einem Sechstel eines Kreisumfangs geöffnet ist. Ein Durchmesser des Teilbereichs, der durch die kreisförmige Kontur begrenzt wird, entspricht der zweiten Erstreckung des Bereichs 18a des Kopplungselements 14a in die zweite Querrichtung 20a.

Das korrespondierende Kopplungselement 15a begrenzt einen Führungskanal 24a zur Führung des Kopplungselements 14a in die teilweise kreisförmige Ausnehmung 23a. An einer Öffnungsstelle des Kreisumfangs der kreisförmigen Kontur in dem zweiten Griffelement 12a mündet der Führungskanal 24a in den durch die geöffnete kreisförmige Kontur begrenzten Teilbereich der teilweise kreisförmigen Ausnehmung 23a. Der Führungskanal 24a kann optional durch Einsetzen eines Verschlusselements, welches entnehmbar oder fest mit dem zweiten Griffelement 12a verbunden ist, verschlossen werden. Eine Breite des Führungskanals 24a entspricht der ersten Erstreckung des Bereichs 18a des Kopplungselements 14a in die erste Querrichtung 19a. Das zweite Griffelement 12a weist zusätzlich zu der teilweise kreisförmigen Ausnehmung 23a eine als Bohrung ausgeführte Öffnung 38a auf, die für den Einsatz eines Teflonelements vorgesehen ist, welches ein direktes Reiben des ersten Griffelements 11a und des zweiten Griffelements 12a aufeinander vermeidet.

Das korrespondierende Kopplungselement 15a bildet ein weiteres Zentrierelement 17a aus. Das als Zentrierelement 16a ausgeführte Kopplungselement 14a und das als Zentrierelement 17a ausgeführte korrespondierende Kopplungselement 15a stellen die einzigen Elemente der Handgriffvorrichtung 10a dar, die die Formschlussverbindung eingehen. Das weitere Zentrierelement 17a führt während einer Drehung des Scherengelenks eine Rotation um das Zentrierelement 16a aus. Eine Verschiebung des zweiten Griffelements 12a von der Drehachse des Scherengelenks ist durch den an die zweite Erstreckung des Bereichs 18a des Kopplungselements 14a in die zweite Querrichtung 20a angepassten Durchmesser des Teilbereichs der teilweise kreisförmigen Ausnehmung 23a, der durch die kreisförmige Kontur begrenzt wird, blockiert.

Das Kopplungselement 14a und das korrespondierende Kopplungselement 15a sind zur Herstellung der Formschlussverbindung mittels einer Verschiebebewegung in einer Ebene parallel zu einer Drehebene des Scherengelenks vorgesehen. Für eine Montage der Griffelemente 11a, 12a der Handgriffvorrichtung 10a wird das zweite Griffelement 12a an einem Ende des Führungskanals 24a, der von dem durch die geöffnete kreisförmige Kontur begrenzten Teilbereich der teilweise kreisförmigen Ausnehmung 23a abgewandt ist, an ein abgerundetes Ende des Bereichs 18a des Kopplungselements 14a angesetzt. Mit einer entlang der zweiten Querrichtung 20a verlaufenden Verschiebebewegung, die vollständig innerhalb der parallel zu der Drehebene des Scherengelenks gelegenen Ebene verläuft, wird das zweite Griffelement 12a auf das Kopplungselement 14a aufgeschoben, bis das Kopplungselement 14a vollständig innerhalb des Teilbereichs der teilweise kreisförmigen Ausnehmung 23a, der durch die kreisförmige Kontur begrenzt wird, aufgenommen ist. In dieser Position kann das zweite Griffelement 12a um das Kopplungselement 14a geschwenkt werden. Grundsätzlich können das Kopplungselement 14a und das korrespondierende Kopplungselement 15a zur Herstellung der Formschlussverbindung mittels einer Verschiebebewegung in einer im Wesentlichen zu der Drehebene des Scherengelenks parallelen Ebene vorgesehen sein, welche beispielsweise gegenüber der Drehebene des Scherengelenks um drei Grad zu dem ersten Griffelement 11a geneigt ist, sodass das zweite Griffelement 12a während einer Montage das Kopplungselement 14a untergreift.

Die Handgriffvorrichtung 10a umfasst eine Werkzeugeinheit 25a zur Halterung eines Werkzeugs und eine Sicherungseinheit 27a, die einen Verschlusshebel 28a umfasst, der eine Schwenkachse 29a zur Schwenkung zwischen einer Freigabestellung und einer Befestigungsstellung für die Werkzeugeinheit 25a aufweist, und die zur reversiblen Halterung der Werkzeugeinheit 25a an zumindest einem der Griffelemente 11a, 12a vorgesehen ist. Grundsätzlich kann die Handgriffvorrichtung 10a auch eine fest an dem ersten Griffelement 11a gelagerte Werkzeugeinheit 25a umfassen, wobei auf eine Sicherungseinheit 27a mit einem Verschlusshebel 28a verzichtet wird und das Werkzeug beispielsweise mittels einer Schraube gesichert wird. Auch ist grundsätzlich vorstellbar, dass die Handgriffvorrichtung 10a eine Werkzeugeinheit 25a zur Halterung eines Werkzeugs und eine Sicherungseinheit 27a, die einen Verschlusshebel 28a umfasst, aufweist, aber ein erstes Griffelement 11a und ein zweites Griffelement 12a aufweist, die fest und inseparabel mittels einer Schraube oder einer Niete in einem Scherengelenk verbunden sind.

Der Verschlusshebel 28a ist als Metallplattenbauteil ausgeführt und mittels eines Druckfederknopfs 43a an dem ersten Griffelement 11a gelagert. Die Werkzeugeinheit 25a umfasst einen Schieber 39a, der einen Haken 47a zur Betätigung mittels des zweiten Griffelements 12a aufweist und einen Schaft 26a, der als offene Röhre ausgeführt ist und den Schieber 39a lagert. Ein Anschlag 42a ist an dem Schaft 26a angeschweißt und verhindert eine zu weite Einziehung der Werkzeugeinheit 25a in das erste Griffelement 11a.

Die Sicherungseinheit 27a weist ein Kurvengetriebe 30a mit zwei aufeinander gleitenden Schrägflächen 32a, 34a auf, die bei Schwenkung des Verschlusshebels 28a in die Freigabestellung zur Freigabe der Werkzeugeinheit 25a aneinander abgleiten. Das Kurvengetriebe 30a umfasst einen Stift 44a, der zur Halterung der Werkzeugeinheit 25a vorgesehen ist, einen ersten Ring 31a, der eine erste Schrägfläche 32a aufweist, einen zweiten Ring 33a, der eine zweite Schrägfläche 34a aufweist, eine Druckfeder 45a zur Rückstellung des ersten Rings 31a und einen Befestigungsring 35a. Die Schwenkachse 29a entspricht einer Mittelachse des Stifts 44a. Die Ringe 31a, 33a weisen jeweils an einer Seite eine entlang des Kreisumfangs variierende Höhe auf, an der die jeweiligen Schrägflächen 32a, 34a ausgebildet sind. Die Schrägflächen 32a, 34a der Ringe 31a, 33a sind so ausgeführt, dass sie zueinander korrespondieren. In der Befestigungsstellung sind die Ringe 31a, 33a so mit den Schrägflächen 32a, 34a aneinander gelagert, dass ein Teilbereich des ersten Rings 31a mit einer größten Höhe an einem Teilbereich des zweiten Rings 33a mit einer geringsten Höhe anliegt und die zwei Ringe 31a, 33a sich zu einem gemeinsamen Gesamtring mit einer konstanten Höhe ergänzen. In der Befestigungsstellung drückt der Stift 44a gegen den Schieber 39a und hält somit die Werkzeugeinheit 25a fest.

Der Befestigungsring 35a ist an dem ersten Griffelement 11a mittels Verschweißen befestigt. Zwischen dem Befestigungsring 35a und dem ersten Ring 31a ist die Druckfeder 45a angeordnet. Die Druckfeder 45a ist dazu vorgesehen, den Stift 44a gegen ein versehentliches Herausziehen zu sichern und den ersten Ring 31a in der Befestigungsstellung gegen den zweiten Ring 33a zu drücken. Der erste Ring 31a ist fest mit dem Stift 44a verbunden, sodass er bei Drehung des Stifts 44a mitgedreht wird. Der zweite Ring 33a ist mit dem ersten Griffelement 11a fest verbunden.

Bei Schwenkung des Verschlusshebels 28a in die Freigabestellung wird der Verschlusshebel 28a um die Schwenkachse 29a verschwenkt, wodurch sich der Stift 44a und der erste Ring 31a um die Schwenkachse 29a verdrehen. Der erste Ring 31a wird dadurch gegenüber dem feststehenden zweiten Ring 33a verdreht. Die Schrägflächen 32a, 34a gleiten dadurch aneinander ab und der erste Ring 31a wird von dem zweiten Ring 33a weggedrückt. Die Ringe 31a, 33a weisen weiterhin einen Kontakt an den Schrägflächen 32a, 34a auf, und ein Abstand der von den Schrägflächen 32a, 34a abgewandten Seiten der Ringe 31a, 33a wird vergrößert. Mit dem ersten Ring 31a wird auch der Stift 44a von dem zweiten Ring 33a weggedrückt und der Schieber 39a der Werkzeugeinheit 25a wird freigegeben. In der Freigabestellung ist der erste Ring 31a von dem zweiten Ring 33a entfernt und der Schieber 39a der Werkzeugeinheit 25a freigegeben, sodass die Werkzeugeinheit 25a demontiert werden kann.

Zur Einlegung eines Werkzeugs in die Handgriffvorrichtung 10a wird das Scherengelenk geöffnet, anschließend die Werkzeugeinheit 25a in das erste Griffelement 11a eingeführt, bis der Haken 47a des Schiebers 39a hinter das zweite Griffelement 12a greift und ein Vorsprung 48a des zweiten Griffelements 12a in den Haken 47a einrastet.

Die Werkzeugeinheit 25a zur Halterung eines Werkzeugs umfasst ein mit dem Schieber 39a fest verbundenes und bewegliches Halteelement 40a und den Schaft 26a, in dem der Schieber 39a gelagert ist und der eine Nut 49a zur Führung des Halteelements 40a aufweist. Das Halteelement 40a ist als Blattfeder ausgeführt und ist mit dem Schieber 39a verschweißt. Der Schieber 39a kann über die Führung des als Blattfeder ausgeführten Halteelements 40a in der Nut 49a relativ zu dem Schaft 26a verschoben werden, wird aber durch das als Blattfeder ausgeführte Halteelement 40a an dem Schaft 26a gehalten, sodass eine vollständige Entnahme des Schiebers 39a aus dem Schaft 26a nicht möglich ist. Für eine Reinigung können der Schaft 26a und der Schieber 39a aus dem ersten Griffelement 11a entnommen werden. Nach Entnahme des Schafts 26a und des Schiebers 39a aus dem ersten Griffelement 11a können der Schaft 26a und der Schieber 39a teilweise voneinander getrennt und gereinigt werden, wobei über das als Blattfeder ausgeführte Halteelement 40a der Schieber 39a mit dem Schaft 26a verbunden bleibt. In einer alternativen Ausgestaltung ist beispielsweise vorstellbar, dass das als Blattfeder ausgeführte Halteelement 40a mittels einer Sicherungsschraube an dem Schieber 39a befestigt ist.

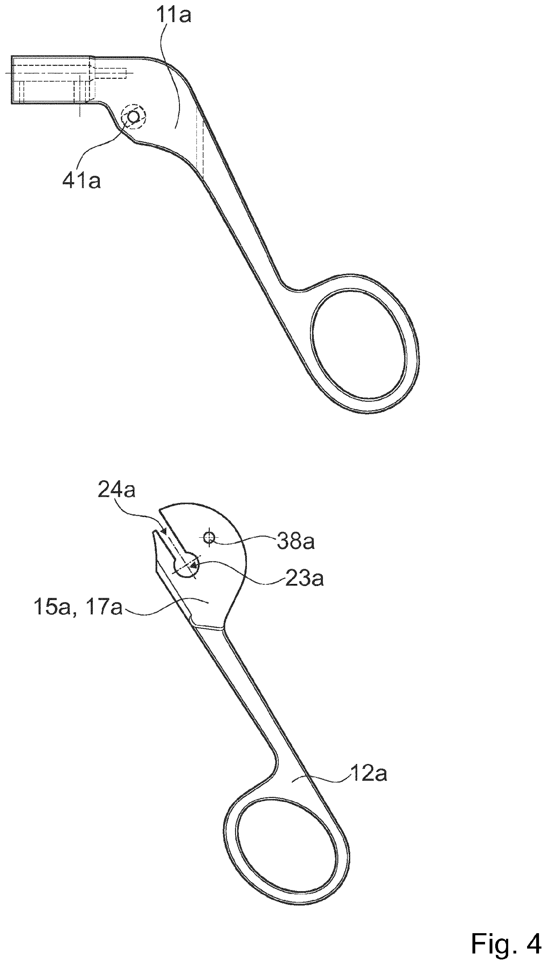

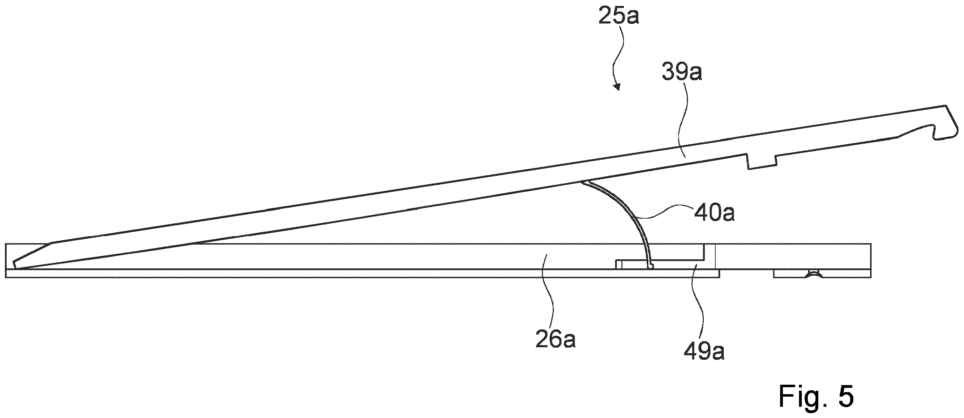

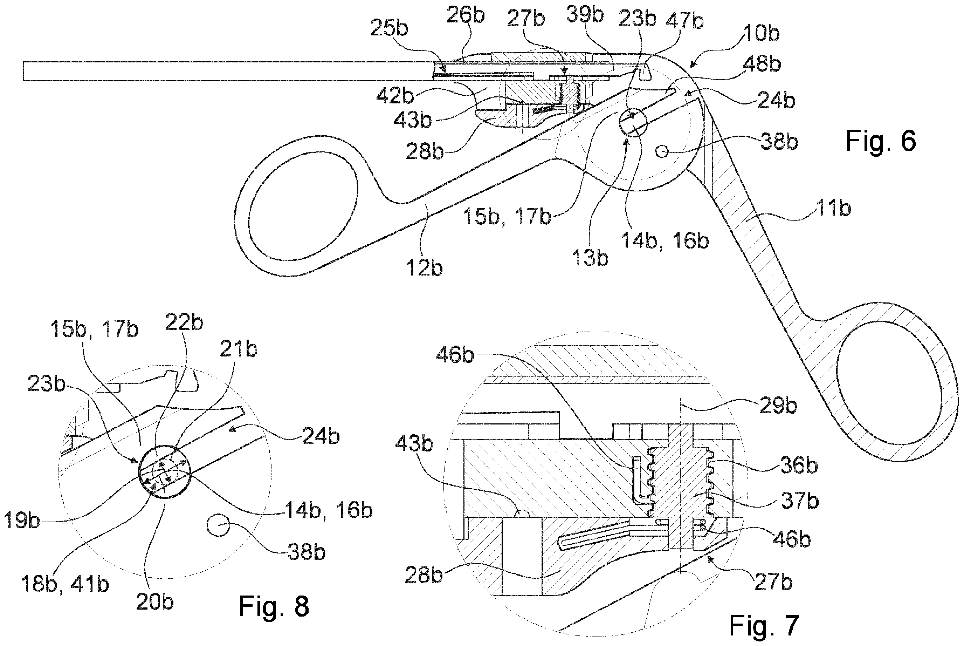

In den Figuren 6 bis 12 sind drei weitere Ausführungsbeispiele gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 bis 5, verwiesen wird. Zur Unterscheidung der Ausführungsbeispiele ist der Buchstabe a den Bezugszeichen des Ausführungsbeispiels in den Figuren 1 bis 5 nachgestellt. In den Ausführungsbeispielen der Figuren 6 bis 12 ist der Buchstabe a durch die Buchstaben b bis d ersetzt.

Die Figuren 6 bis 8 zeigen eine als Chirurgiewerkzeug-Handgriffvorrichtung ausgeführte Handgriffvorrichtung 10b mit einem ersten Griffelement 11b und einem zweiten Griffelement 12b und einem Scherengelenk, mittels dessen die zwei Griffelemente 11b, 12b schwenkbar und separierbar verbunden sind, sowie mit einer Kopplungseinheit 13b, die dazu vorgesehen ist, die beiden Griffelemente 11b, 12b entgegen einer Separierrichtung zusammenzuhalten, und die ein Kopplungselement 14b aufweist, das dazu vorgesehen ist, abhängig von einer Relativposition der Griffelemente 11b, 12b eine Formschlussverbindung derselben entgegen der Separierrichtung herzustellen. Das Kopplungselement 14b ist als Zentrierelement 16b zur Zentrierung der Formschlussverbindung ausgebildet. Das Kopplungselement 14b sowie ein korrespondierendes Kopplungselement 15b der Kopplungseinheit 13b, welches eine zumindest teilweise kreisförmige Ausnehmung 23b zur Aufnahme des Kopplungselements 14b begrenzt, sind identisch zu dem vorherigen Ausführungsbeispiel ausgeführt.

Die Handgriffvorrichtung 10b umfasst eine Werkzeugeinheit 25b zur Halterung eines Werkzeugs und eine Sicherungseinheit 27b, die einen Verschlusshebel 28b umfasst, der eine Schwenkachse 29b zur Schwenkung zwischen einer Freigabestellung und einer Befestigungsstellung für die Werkzeugeinheit 25b aufweist, und die zur reversiblen Halterung der Werkzeugeinheit 25b an zumindest einem der Griffelemente 11b, 12b vorgesehen ist. Der Verschlusshebel 28b ist als Metallplattenbauteil ausgeführt und mittels eines Druckfederknopfs 43b an dem ersten Griffelement 11b gelagert. Die Werkzeugeinheit 25b umfasst einen Schieber 39b, der einen Haken 47b zur Betätigung mittels des zweiten Griffelements 12b aufweist, und einen Schaft 26b, der als offene Röhre ausgeführt ist und den Schieber 39b lagert. Ein Anschlag 42b ist an dem Schaft 26b angeschweißt und verhindert eine zu weite Einziehung der Werkzeugeinheit 25b in das erste Griffelement 11b.

Die Sicherungseinheit 27b weist ein Gewinde 36b und eine Gewindeschraube 37b, die bei Schwenkung des Verschlusshebels 28b aus dem Gewinde 36b ausgeschraubt wird und die Werkzeugeinheit 25b freigibt, auf. Das Gewinde 36b ist in dem ersten Griffelement 11b ausgeführt. Das Gewinde 36b und die Gewindeschraube 37b sind als Trapezgewinde ausgeführt. Die Gewindeschraube 37b ist in der Befestigungsstellung vollständig in dem Gewinde 36b aufgenommen und drückt mit einem Endvorsprung gegen den Schieber 39b und hält dadurch die Werkzeugeinheit 25b. Ein zweiter Endvorsprung ist fest mit dem Verschlusshebel 28b verbunden. Die Schwenkachse 29b verläuft zentral durch die Gewindeschraube 37b. Bei einer Schwenkung des Verschlusshebels 28b wird die Gewindeschraube 37b gedreht und dreht sich um eine Viertelumdrehung aus dem Gewinde 36b heraus, wodurch der Endvorsprung von dem Schieber 39b entfernt wird und die Werkzeugeinheit 25b freigegeben wird.

Eine Drehfeder 46b ist in einer ausgefrästen Aufnahme, die sich um das Gewinde 36b erstreckt und die in dem ersten Griffelement 11b und dem Verschlusshebel 28b ausgeführt ist, aufgenommen. Die Drehfeder 46b kontaktiert die Gewindeschraube 37b. Bei Schwenkung des Verschlusshebels 28b um die Schwenkachse 29b wird die Drehfeder 46b durch die sich drehende Gewindeschraube 37b gespannt. Bei Rückschwenkung des Verschlusshebels 28b in die Befestigungsstellung übt die Drehfeder 46b Druck auf die Gewindeschraube 37b aus und schraubt die Gewindeschraube 37b wieder vollständig in das Gewinde 36b ein.

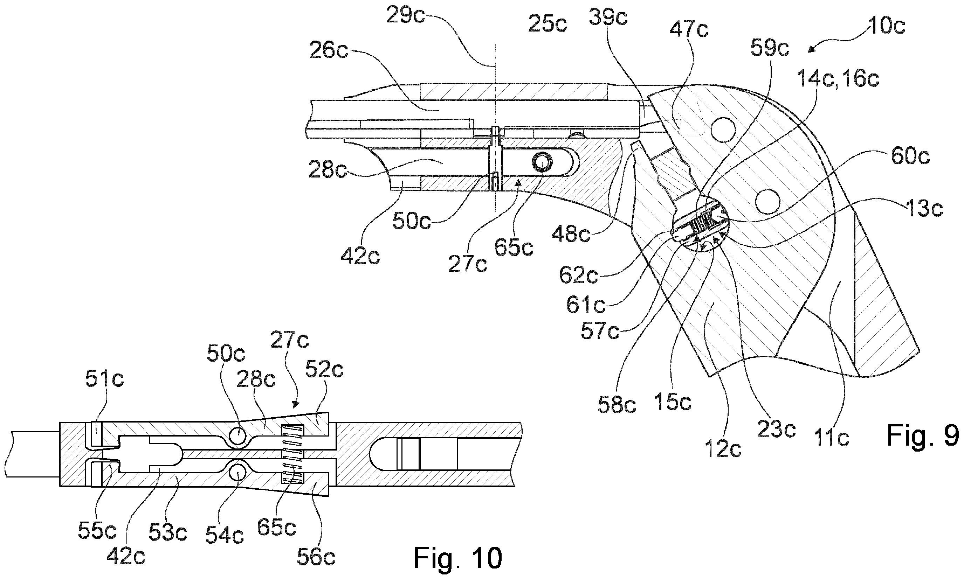

Die Figuren 9 und 10 zeigen einen Ausschnitt einer als als Chirurgiewerkzeug-Handgriffvorrichtung ausgeführten Handgriffvorrichtung 10c mit einem ersten Griffelement 11c und einem zweiten Griffelement 12c und einem Scherengelenk, mittels dessen die zwei Griffelemente 11c, 12c schwenkbar und separierbar verbunden sind, sowie mit einer Kopplungseinheit 13c, die dazu vorgesehen ist, die beiden Griffelemente 11c, 12c entgegen einer Separierrichtung zusammenzuhalten, und die ein Kopplungselement 14c aufweist, das dazu vorgesehen ist, abhängig von einer Relativposition der Griffelemente 11c, 12c eine Formschlussverbindung derselben entgegen der Separierrichtung herzustellen. Das Kopplungselement 14c ist als Zentrierelement 16c zur Zentrierung der Formschlussverbindung ausgebildet. Die Handgriffvorrichtung 10c weist ein korrespondierendes Kopplungselement 15c der Kopplungseinheit 13c auf, welches eine teilweise kreisförmige Ausnehmung 23c zur Aufnahme des Kopplungselements 14c begrenzt. Das korrespondierende Kopplungselement 15c ist als Teilbereich des zweiten Griffelements 12c ausgeführt, der die teilweise kreisförmige Ausnehmung 23c aufweist.

Die Handgriffvorrichtung 10c umfasst eine Werkzeugeinheit 25c zur Halterung eines Werkzeugs und eine Sicherungseinheit 27c, die einen Verschlusshebel 28c umfasst, der eine Schwenkachse 29c zur Schwenkung zwischen einer Freigabestellung und einer Befestigungsstellung für die Werkzeugeinheit 25c aufweist, und die zur reversiblen Halterung der Werkzeugeinheit 25c an zumindest einem der Griffelemente 11c, 12c vorgesehen ist. Der Verschlusshebel 28c ist als Metallplattenbauteil ausgeführt. Die Werkzeugeinheit 25c umfasst einen Schieber 39c, der einen Haken 47c zur Betätigung mittels des zweiten Griffelements 12c aufweist, und einen Schaft 26c, der als offene Röhre ausgeführt ist und den Schieber 39c lagert. Das zweite Griffelement 12c weist einen Vorsprung 48c auf, der zum Einrasten in den Haken 47c des Schiebers 39c und zur Kopplung des Schiebers 39c und des zweiten Griffelements 12c vorgesehen ist. Der Schaft 26c ist zudem für eine Aufnahme des Werkzeugs vorgesehen. Die Handgriffvorrichtung 10c weist einen Anschlag 42c auf, der abnehmbar an dem ersten Griffelement 11c befestigt ist und der dazu vorgesehen ist, eine zu weite Einziehung der Werkzeugeinheit 25c in das erste Griffelement 11c zu verhindern.

Der Verschlusshebel 28c befestigt den Anschlag 42c direkt an dem ersten Griffelement 11c in der Befestigungsstellung. Der Verschlusshebel 28c ist mittels eines Verriegelungsbolzens 50c an dem ersten Griffelement 11c befestigt. In der Freigabestellung ist der Verschlusshebel 28c für eine Freigabe des Schafts 26c vorgesehen. Der Verschlusshebel 28c ist um eine Achse des Verriegelungsbolzens 50c schwenkbar. Der Verschlusshebel 28c umfasst eine vorspringende Kante 51c, die in der Befestigungsstellung an einer Vorderseite des Anschlags 42c angreift und den Anschlag 42c gegen das erste Griffelement 11c sichert (Fig. 10). Der Verschlusshebel 28c weist einen Betätigungsbereich 52c auf, der von der vorspringenden Kante 51c abgewandt ist. Durch Druck auf den Betätigungsbereich 52c des Verschlusshebels 28c wird der Verschlusshebel 28c um den Verriegelungsbolzen 50c geschwenkt und die vorspringende Kante 51c von dem Anschlag 42c abgehoben, sodass der Anschlag 42c demontiert werden kann. Nach Demontage des Anschlags 42c können der Schaft 26c und weitere Bestandteile der Werkzeugeinheit 25c demontiert werden.

Die Sicherungseinheit 27c der Handgriffvorrichtung 10c umfasst einen weiteren Verschlusshebel 53c, der an einer dem Verschlusshebel 28c abgewandten Seite des ersten Griffelements 11c angeordnet ist und der den Anschlag 42c direkt an dem ersten Griffelement 11c in der Befestigungsstellung befestigt (Fig. 10). Ein Verriegelungsbolzen 54c sichert den weiteren Verschlusshebel 53c an dem ersten Griffelement 11c. Der weitere Verschlusshebel 53c ist identisch zu dem ersten Verschlusshebel 28c ausgeführt und weist ebenfalls eine vorspringende Kante 55c und einen Betätigungsbereich 56c auf. Durch Druck auf den Betätigungsbereich 56c wird der weitere Verschlusshebel 53c um den Verriegelungsbolzen 54c geschwenkt und gibt den Anschlag 42c frei. Zur Demontage der Werkzeugeinheit 25c werden durch beidseitigen Druck an der Handgriffvorrichtung 10c die Betätigungsbereiche 52c, 56c betätigt und die Verschlusshebel 28c, 53c um die Verriegelungsbolzen 50c, 54c in die Freigabestellungen geschwenkt, sodass der Anschlag 42c und weitere Teile der Werkzeugeinheit 25c demontiert werden können. Zur vollständigen Zerlegung der Handgriffvorrichtung 10c können anschließend die Griffelemente 11c, 12c voneinander getrennt werden. Die Sicherungseinheit 27c umfasst eine Feder 65c, die zwischen den beiden Verschlusshebeln 28c, 53c angeordnet ist und die an den Betätigungsbereichen 52c, 56c angreift. Die Feder 65c sichert die Verschlusshebel 28c, 53c in der Befestigungsstellung.

Das Kopplungselement 14c und das korrespondierende Kopplungselement 15c weisen eine Arretierungseinheit 57c auf, die dazu vorgesehen ist, die Griffelemente 11c, 12c in einer Arretierungsstellung zu arretieren (Fig. 10). In der Arretierungsstellung ist sichergestellt, dass der Vorsprung 48c des zweiten Griffelements 12c in den Haken 47c des Schiebers 39c eingreifen kann und somit eine erfolgreiche Kopplung des Schiebers 39c an das zweite Griffelement 12c erfolgen kann. Die Arretierungseinheit 57c umfasst ein Druckstück 58c aus einer Druckfeder 59c, einem Gewindestift 60c und einem Kugelbolzen 61c, der einen halbkugelförmigen Kopf mit einem Durchmesser von 1,2 cm aufweist. Die Druckfeder 59c ist als Schraubenfeder mit einer Länge von 4 cm, einem äußeren Durchmesser von 1,5 cm und einem inneren Durchmesser von 1,1 cm ausgeführt. Das Druckstück 58c ist in dem Kopplungselement 14c aufgenommen. Die Arretierungseinheit 57c umfasst eine halbkugelförmige Ausnehmung 62c an dem korrespondierenden Kopplungselement 15c, die zur Aufnahme des halbkugelförmigen Kopfs des Kugelbolzens 61c vorgesehen ist.

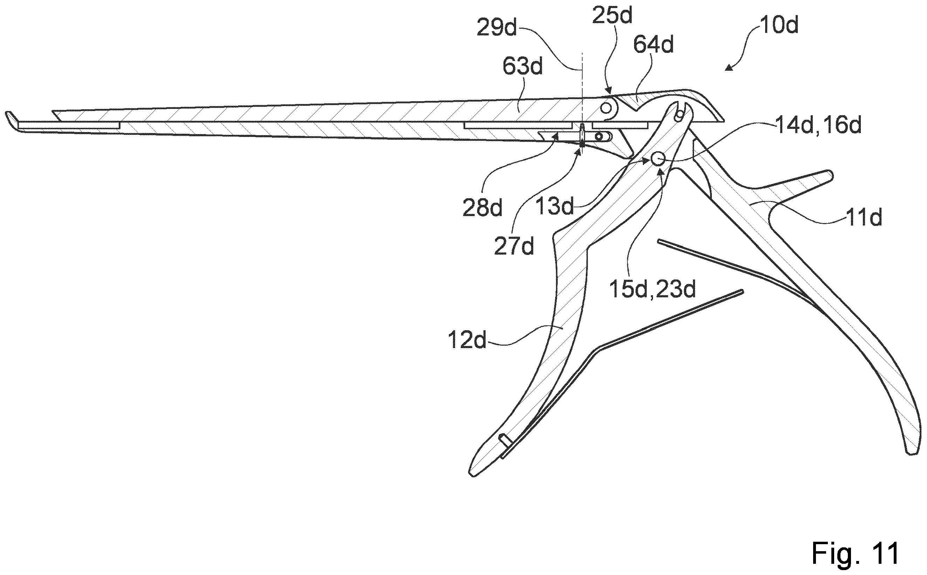

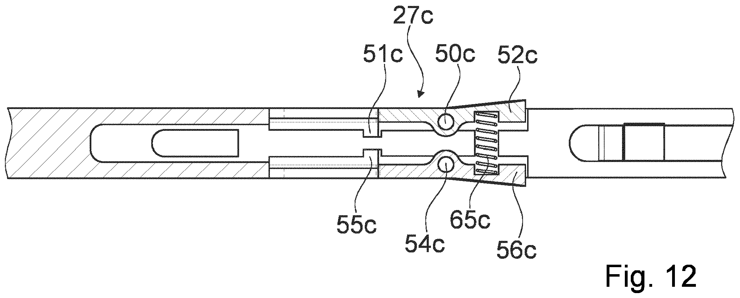

In den Figuren 11 und 12 ist ein weiteres Ausführungsbeispiel einer als Chirurgiewerkzeug-Handgriffvorrichtung ausgeführten Handgriffvorrichtung 10d dargestellt. Die Handgriffvorrichtung 10d ist als eine Kerrison-Stanze ausgeführt, mit der beispielsweise menschliches Gewebe, beispielsweise Rückenmark, ausgestanzt werden kann. Die Handgriffvorrichtung 10d umfasst ein erstes Griffelement 11d und ein zweites Griffelement 12d und ein Scherengelenk, mittels dessen die zwei Griffelemente 11d, 12d schwenkbar und separierbar verbunden sind. Die Handgriffvorrichtung 10d umfasst zudem eine Kopplungseinheit 13d, die dazu vorgesehen ist, die beiden Griffelemente 11d, 12d entgegen einer Separierrichtung zusammenzuhalten, und die ein Kopplungselement 14d aufweist, das dazu vorgesehen ist, abhängig von einer Relativposition der Griffelemente 11d, 12d eine Formschlussverbindung derselben entgegen der Separierrichtung herzustellen. Das Kopplungselement 14d ist als Zentrierelement 16d zur Zentrierung der Formschlussverbindung ausgebildet. Die Handgriffvorrichtung 10d weist ein korrespondierendes Kopplungselement 15d der Kopplungseinheit 13d auf, welches eine teilweise kreisförmige Ausnehmung 23d zur Aufnahme des Kopplungselements 14d begrenzt. Das korrespondierende Kopplungselement 15d ist als Teilbereich des zweiten Griffelements 12d ausgeführt, der die teilweise kreisförmige Ausnehmung 23d aufweist.

Die Handgriffvorrichtung 10d umfasst eine Werkzeugeinheit 25d zur Halterung eines Werkzeugs und eine Sicherungseinheit 27d, die einen Verschlusshebel 28d umfasst, der eine Schwenkachse 29d zur Schwenkung zwischen einer Freigabestellung und einer Befestigungsstellung für die Werkzeugeinheit 25d aufweist, und die zur reversiblen Halterung der Werkzeugeinheit 25d an zumindest einem der Griffelemente 11d, 12d vorgesehen ist. Der Verschlusshebel 28d ist als Metallplattenbauteil ausgeführt. Die Werkzeugeinheit 25d umfasst zwei Aufsätze 63d, 64d, die mit dem ersten Griffelement 11d ein Werkzeug ausbilden. Der erste Aufsatz 63d ist in Bezug auf eine Stanzkante des Werkzeugs vor dem zweiten Aufsatz 64d angeordnet. In einem Ausstanzvorgang wird menschliches Gewebe zwischen dem ersten Aufsatz 63d und dem ersten Griffelement 11d ergriffen und ausgestanzt. Der zweite Aufsatz 64d ist gegenüber dem ersten Aufsatz 63d in Richtung auf einen Betätiger der Handgriffvorrichtung 10d versetzt.

Der Verschlusshebel 28d ist mittels eines Verriegelungsbolzens 50d an dem ersten Griffelement 11d befestigt. In der Befestigungsstellung befestigt der Verschlusshebel 28d den ersten Aufsatz 63d direkt an dem ersten Griffelement 11d. In der Freigabestellung ist der Verschlusshebel 28d für eine Freigabe des ersten Aufsatzes 63d vorgesehen. Der Verschlusshebel 28d ist um eine Achse des Verriegelungsbolzens 50d schwenkbar. Der Verschlusshebel 28d umfasst eine vorspringende Kante 51d, die in der Befestigungsstellung in den ersten Aufsatz 63d eingreift und den ersten Aufsatz 63d an dem ersten Griffelement 11d sichert. Auf einer von der vorspringenden Kante 51d abgewandten Seite weist der Verschlusshebel 28d einen Betätigungsbereich 52d auf, der aus einer Ebene des ersten Griffelements 11d hervorsteht. Durch Druck auf den Betätigungsbereich 52d in Richtung auf das erste Griffelement 11d zu wird der Verschlusshebel 28d um den Verriegelungsbolzen 50d geschwenkt und die vorspringende Kante 51d von ersten Aufsatz 63d abgehoben, sodass der ersten Aufsatz 63d demontiert werden kann. Nach Demontage des ersten Aufsatzes 63d kann der zweite Aufsatz 64d demontiert werden.

Die Sicherungseinheit 27d der Handgriffvorrichtung 10d umfasst einen weiteren Verschlusshebel 53d, der an einer dem Verschlusshebel 28d abgewandten Seite des ersten Griffelements 11d angeordnet ist und der den ersten Aufsatz 63d direkt an dem ersten Griffelement 11d in der Befestigungsstellung befestigt. Ein Verriegelungsbolzen 54d sichert den weiteren Verschlusshebel 53d an dem ersten Griffelement 11d. Der weitere Verschlusshebel 53d ist identisch zu dem ersten Verschlusshebel 28d ausgeführt und weist ebenfalls eine vorspringende Kante 55d und einen Betätigungsbereich 56d auf. Durch Druck auf den Betätigungsbereich 56d wird der weitere Verschlusshebel 53d um den Verriegelungsbolzen 54d geschwenkt und gibt den ersten Aufsatz 63d frei. Zur Demontage der Werkzeugeinheit 25d werden durch beidseitigen Druck an der Handgriffvorrichtung 10d die Betätigungsbereiche 52d, 56d betätigt und die Verschlusshebel 28d, 53d um die Verriegelungsbolzen 50d, 54d in die Freigabestellungen geschwenkt, sodass der erste Aufsatz 63d und anschließend der zweite Aufsatz 64d demontiert werden können. Zur vollständigen Zerlegung der Handgriffvorrichtung 10d können anschließend die Griffelemente 11d, 12d voneinander getrennt werden.

## Patentansprüche

1. Handgriffvorrichtung, insbesondere Chirurgiewerkzeug-Handgriffvorrichtung, mit einem ersten Griffelement (11a; 11b; 11c; 11d) und einem zweiten Griffelement (12a; 12b; 12c; 12d), mit einer Werkzeugeinheit (25a; 25b; 25c; 25d) zur Halterung eines Werkzeugs, und einem Scherengelenk, mittels dessen die zwei Griffelemente (11a; 11b; 11c; 11d, 12a; 12b; 12c; 12d) schwenkbar und separierbar verbunden sind, sowie mit einer Kopplungseinheit (13a; 13b; 13c; 13d), die dazu vorgesehen ist, die beiden Griffelemente (11a; 11b; 11c; 11d, 12a; 12b; 12c; 12d) entgegen einer Separierrichtung zusammenzuhalten, und die wenigstens ein Kopplungselement (14a; 14b; 14c; 14d) aufweist, das dazu vorgesehen ist, abhängig von einer Relativposition der Griffelemente (11a; 11b; 11c; 11d, 12a; 12b; 12c; 12d) eine Formschlussverbindung derselben entgegen der Separierrichtung herzustellen, wobei das wenigstens eine Kopplungselement (14a; 14b; 14c; 14d) als Zentrierelement (16a; 16b; 16c; 16d) zur Zentrierung der Formschlussverbindung ausgebildet ist, wobei das wenigstens eine Kopplungselement (14a; 14b) zumindest einen Bereich (18a; 18b) aufweist, der in einer ersten Querrichtung (19a; 19b) eine erste Erstreckung und in einer zweiten Querrichtung (20a; 20b) eine zweite Erstreckung aufweist, die größer ist als die erste Erstreckung, mit zumindest einem korrespondierenden Kopplungselement (15a; 15b; 15c; 15d), welches eine zumindest teilweise kreisförmige Ausnehmung (23a; 23b; 23c; 23d) zur Aufnahme des Kopplungselements (14a; 14b; 14c; 14d) begrenzt, wobei das Kopplungselement (14a; 14b) und das korrespondierende Kopplungselement (15a; 15b) zur Herstellung der Formschlussverbindung mittels einer Verschiebebewegung in einer Ebene zumindest im Wesentlichen parallel zu einer Drehebene des Scherengelenks vorgesehen sind,
**dadurch gekennzeichnet, dass**
das Kopplungselement (14a; 14b) in einer Richtung senkrecht zu der ersten Querrichtung (19a; 19b) und der zweiten Querrichtung (20a; 20b) an einem Endstück eine den Bereich (18a; 18b) überstehende Krempe (22a) mit einem kreisförmigen Querschnitt aufweist, wobei an einem von der Krempe (22a) abgewandten Endstück des Kopplungselements (14a; 14b) das Kopplungselement (14a; 14b) einen Einsteckpin (21a) aufweist, der zum Einstecken in eine korrespondierende Bohrung des ersten Griffelements (11a; 11b; 11c; 11d) vorgesehen ist, wobei die Bohrung in einem Zentrum einer rechteckigen Ausfräsung (41a) des ersten Griffelements (11a; 11b) ausgeführt ist, wobei die rechteckige Ausfräsung (41a; 41b) in äußeren Abmessungen einem Querschnitt des Bereichs (18a; 18b) des Kopplungselements (14a; 14b) mit den Querrichtungen (19a; 19b, 20a; 20b) entspricht, wobei das korrespondierende Kopplungselement (15a; 15b; 15c; 15d) als Teilbereich des zweiten Griffelements (12a; 12b; 12c; 12d) ausgeführt ist, der die teilweise kreisförmige Ausnehmung (23a; 23b; 23c; 23d) aufweist.

2. Handgriffvorrichtung nach Anspruch 1,
**dadurch gekennzeichnet, dass**
das wenigstens eine Kopplungselement (14a; 14b) an Enden des Bereichs (18a; 18b) abgerundet ausgeführt ist.

3. Handgriffvorrichtung nach einem der vorhergehenden Ansprüche,
**dadurch gekennzeichnet, dass**
das korrespondierende Kopplungselement (15a; 15b) einen Führungskanal (24a; 24b) zur Führung des Kopplungselements (14a; 14b) in die zumindest teilweise kreisförmige Ausnehmung (23a; 23b) begrenzt.

4. Handgriffvorrichtung nach einem der vorhergehenden Ansprüche,
**dadurch gekennzeichnet, dass**
das korrespondierende Kopplungselement (15a; 15b) ein weiteres Zentrierelement (17a; 17b) ausbildet.

5. Handgriffvorrichtung nach einem der vorhergehenden Ansprüche,
**dadurch gekennzeichnet, dass**
das Kopplungselement (14c) und das korrespondierende Kopplungselement (15c) zumindest eine Arretierungseinheit (57c) aufweisen, die dazu vorgesehen ist, die Griffelemente (11c, 12c) in zumindest einer Arretierungsstellung zu arretieren.

6. Handgriffvorrichtung nach einem der vorhergehenden Ansprüche, mit einer Sicherungseinheit (27a; 27b; 27c; 27d), die einen Verschlusshebel (28a; 28b; 28c; 28d) umfasst, der eine Schwenkachse (29a; 29b; 29c; 29d) zur Schwenkung zwischen einer Freigabestellung und einer Befestigungsstellung für die Werkzeugeinheit (25a; 25b; 25c; 25d) aufweist, und die zur reversiblen Halterung der Werkzeugeinheit (25a; 25b; 25c; 25d) an zumindest einem der Griffelemente (11a; 11b; 11c; 11d, 12a; 12b; 12c; 12d) vorgesehen ist,
**dadurch gekennzeichnet, dass**
die Sicherungseinheit (27a) ein Kurvengetriebe (30a) mit zwei aufeinander gleitenden Schrägflächen (32a, 34a) aufweist, die bei Schwenkung des Verschlusshebels (28a) in die Freigabestellung zur Freigabe der Werkzeugeinheit (25a) aneinander abgleiten.

7. Handgriffvorrichtung nach Anspruch 6,
**dadurch gekennzeichnet, dass**
die Sicherungseinheit (27b) ein Gewinde (36b) und eine Gewindeschraube (37b), die bei Schwenkung des Verschlusshebels (28b) aus dem Gewinde (36b) ausgeschraubt wird und die Werkzeugeinheit (25b) freigibt, aufweist.

8. Handgriffvorrichtung nach Anspruch 6 oder 7,
**dadurch gekennzeichnet, dass**
die Werkzeugeinheit (25c) einen Schaft (26c) zur Aufnahme eines Werkzeugs und einen Anschlag (42c) umfasst, an dem der Schaft (26c) gelagert ist und der Verschlusshebel (28c) zur direkten Befestigung des Anschlags (42c) an einem der Griffelemente (11c, 12d) in der Befestigungsstellung und für eine Freigabe des Anschlags (42c) in der Freigabestellung vorgesehen ist.

9. Handgriffvorrichtung nach einem der Ansprüche 6 bis 8,
**dadurch gekennzeichnet, dass**
die Werkzeugeinheit (25d) zumindest einen Aufsatz (63d, 64d) umfasst, der mit dem ersten Griffelement (11d) ein Werkzeug ausbildet und der in der Befestigungsstellung von dem Verschlusshebel (28d) an dem ersten Griffelement (11d) befestigt wird.

10. Handgriffvorrichtung nach Anspruch einem der Ansprüche 6 bis 9,
**dadurch gekennzeichnet, dass**
die Sicherungseinheit (27c; 27d) einen weiteren Verschlusshebel (53c; 53d) umfasst.

11. System mit einer Handgriffvorrichtung (10a; 10b; 10c; 10d) nach einem der vorhergehenden Ansprüche und einem Werkzeug, insbesondere einem Chirurgiewerkzeug.

## Claims

1. Handle device, in particular surgical-tool handle device,
with a first handle element (11a; 11b; 11c; 11d) and a second handle element (12a; 12b; 12c; 12d),
with a tool unit (25a; 25b; 25c; 25d) for a holding of a tool,
and with a shear joint by means of which the two handle elements (11a; 11b; 11c; 11d, 12a; 12b; 12c; 12d) are connected pivotably and separably, and with a coupling unit (13a; 13b; 13c; 13d), which is configured to keep the two handle elements (11a; 11b; 11c; 11d, 12a; 12b; 12c; 12d) together counter to a separation direction and which comprises at least one coupling element (14a; 14b; 14c; 14d) that is configured to establish, depending on a relative position of the handle elements (11a; 11b; 11c; 11d, 12a; 12b; 12c; 12d), a form-fit connection of the handle elements (11a; 11b; 11c; 11d, 12a; 12b; 12c; 12d) counter to the separation direction, the at least one coupling element (14a; 14b; 14c; 14d) being embodied as a centering element (16a; 16b; 16c; 16d) for a centering of the form-fit connection,
wherein the at least one coupling element (14a; 14b; 14c; 14d) comprises at least one region (18a; 18b) which has, in a first transverse direction (19a; 19b), a first extension and has, in a second transverse direction (20a; 20b), a second extension which is greater than the first extension,
with at least one corresponding coupling element (15a; 15b; 15c; 15d), which delimits an at least partly circle-shaped recess (23a; 23b; 23c; 23d) for accommodating the coupling element (14a; 14b; 14c; 14d),
wherein the coupling element (14a; 14b) and the corresponding coupling element (15a; 15b) are configured for establishing the form-fit connection via a translational movement in a plane at least substantially parallel to a rotary plane of the shear joint,
**characterized in that**
the coupling element (14a; 14b) comprises in a direction perpendicular to the first transverse direction (19a; 19b) and to the second transverse direction (20a; 20b), at an end piece, a flange (22a) protruding over the region (18a; 18b) and having a circle-shaped cross-section,
wherein at an end piece of the coupling element (14a; 14b) that faces away from the flange (22a), the coupling element (14a; 14b) comprises a plug-in pin (21a), which is configured to be stuck into a corresponding bore of the first handle element (11a; 11b; 11c; 11d),
the bore being realized in a centre of a rectangular milled-out portion (41a) of the first handle element (11a; 11b),
the rectangular milled-out portion (41a) corresponding in its outer dimensions to a cross-section of the region (18a; 18b) of the coupling element (14a; 14b) with the transverse directions (19a; 19b; 20a; 20b),
wherein the corresponding coupling element (15a; 15b; 15c; 15d) is embodied as a partial region of the second handle element (12a; 12b; 12c; 12d), which has the partly circle-shaped recess (23a; 23b; 23c; 23d).

2. Handle device according to claim 1,
**characterised in that** the at least one coupling element (14a; 14b) is realized in such a way that it is rounded off at ends of the region (18a; 18b).

3. Handle device according to one of the preceding claims,
**characterised in that** the corresponding coupling element (15a; 15b) delimits a guiding channel (24a; 24b) for guiding the coupling element (14a; 14b) into the at least partly circle-shaped recess (23a; 23b).

4. Handle device according to one of the preceding claims,
**characterised in that** the corresponding coupling element (15a; 15b) forms a further centering element (17a; 17b).

5. Handle device according to one of the preceding claims,
**characterised in that** the coupling element (14c) and the corresponding coupling element (15c) comprise at least one blocking unit (57c), which is configured for blocking the handle elements (11c, 12c) in at least one blocking position.

6. Handle device according to one of the preceding claims,
with a securing unit (27a; 27b, 27c, 27d) comprising a locking lever (28a; 28b; 28c; 28d) which has a pivot axis (29a; 29b; 29c; 29d) for pivoting between a release position and a fixing position for the tool unit (25a; 25b; 25c; 25d), the securing unit (27a; 27b; 27c; 27d) being configured for reversibly holding the tool unit (25a; 25b; 25c; 25d) on at least one of the handle elements (11a; 11b; 11c; 11d, 12a; 12b; 12c; 12d),
**characterised in that** the securing unit (27a) comprises a cam mechanism (30a) with two inclined planes (32a, 34a) sliding on one another, which slide off one another to release the tool unit (25a) when the locking lever (28a) is pivoted into the release position.

7. Handle device according to claim 6,
**characterised in that** the securing unit (27b) comprises a thread (36b) and a thread screw (37b), which is screwed out of the thread (36b) and releases the tool unit (25b) when the locking lever (28b) is pivoted.

8. Handle device according to claim 6 or 7,
**characterised in that** the tool unit (25c) comprises a shaft (26c) for accommodating a tool, and comprises a stop (42c) on which the shaft (26c) is supported,
and that the locking lever (28c) is configured to directly fasten the stop (42c) on one of the handle elements (11c; 12d) in the fixing position and to release the stop (42c) in the release position.

9. Handle device according to one of claims 6 to 8,
**characterised in that** the tool unit (25d) comprises at least one upper attachment (63d, 64d), which forms a tool together with the first handle element (11d) and which is in the fixing position fastened to the first handle element (11d) by the locking lever (28d).

10. Handle device according to one of claims 6 to 9,
**characterised in that** securing unit (27c; 27d) comprises another locking lever (53c; 53d).

11. System with a handle device (10a; 10b; 10c; 10d) according to one of the preceding claims and with a tool, in particular a surgical tool.

## Revendications

1. Dispositif de poignée, en particulier dispositif de poignée d'outil chirurgical, avec un premier élément de poignée (11a ; 11b ; 11c ; 11d) et un deuxième élément de poignée (12a ; 12b ; 12c ; 12d),
avec une unité-outil (25a ; 25b ; 25c ; 25d) pour la retenue d'un outil, et avec une articulation à ciseaux moyennant laquelle les deux éléments de poignée (11a ; 11b ; 11c; 11d ; 12a ; 12b ; 12c ; 12d) sont liés de façon pivotable et séparable, et
avec une unité de couplage (13a ; 13b ; 13c ; 13d) qui est destinée à tenir ensemble les deux éléments de poignée (11a ; 11b ; 11c ; 11d ; 12a ; 12b ; 12c ; 12d) contre une direction de séparation, et comprend au moins un élément de couplage (14a ; 14b ; 14c ; 14d) conçu pour créer - indépendamment d'une position relative des éléments de poignée (11a ; 11b ; 11c ; 11d ; 12a ; 12b ; 12c ; 12d) - une liaison en forme desdits éléments de poignée (11a ; 11b ; 11c ; 11d ; 12a ; 12b ; 12c ; 12d) contre la direction de séparation,
l'au moins un élément de couplage (14a ; 14b ; 14c ; 14d) étant réalisé comme élément de centrage (16a ; 16b ; 16c ; 16d) pour le centrage de la liaison en forme,
où l'au moins un élément de couplage (14a ; 14b) comprend au moins une zone (18a ; 18b) ayant dans une première direction transversale (19a ; 19b) une première étendue et dans une deuxième direction transversale (20a ; 20b) une deuxième étendue supérieure à la première étendue,
avec au moins un élément de couplage correspondant (15a ; 15b ; 15c ; 15d) délimitant une échancrure (23a ; 23b ; 23c ; 23d), qui est au moins partiellement en forme de cercle, pour recevoir l'élément de couplage (14a ; 14b ; 14c ; 14d), l'élément de couplage (14a ; 14b) et l'élément de couplage correspondant (15a ; 15b) étant destinés à créer la liaison en forme par le biais d'un mouvement déplaçant dans un plan au moins sensiblement parallèle à un plan rotatif de l'articulation à ciseaux,
**caractérisé en ce que**
l'élément de couplage (14a ; 14b) comprend sur une pièce finale, dans une direction perpendiculaire à la première direction transversale (19a ; 19b) et à la deuxième direction transversale (20a ; 20b), un rebord (22a) saillant au-delà de la zone (18a ; 18b) et ayant une coupe transversale en forme de cercle, où sur une pièce finale de l'élément de couplage (14a ; 14b) détournée du rebord (22a), l'élément de couplage (14a ; 14b) comprend une broche à enfichage (21a) conçu pour être enfiché dans un alésage correspondant du premier élément de poignée (11a ; 11b ; 11c; 11d),
l'alésage étant réalisé dans le centre d'une fraisure rectangulaire (41a) du premier élément de poignée (11a ; 11b),
la fraisure rectangulaire (41a ; 41b) correspondant en ses dimensions extérieures à une coupe transversale de la zone (18a ; 18b) de l'élément de couplage (14a ; 14b) avec les directions transversales (19a ; 19b ; 20a ; 20b),
où l'élément de couplage correspondant (15a ; 15b ; 15c ; 15d) est réalisé comme zone partielle du deuxième élément de poignée (12a ; 12b ; 12c ; 12d) ayant l'échancrure (23a ; 23b ; 23c ; 23d) qui est partiellement en forme de cercle.

2. Dispositif de poignée selon la revendication 1,
**caractérisé en ce que** l'au moins un élément de couplage (14a ; 14b) est réalisé aux extrémités de la zone (18a ; 18b) en forme arrondie.

3. Dispositif de poignée selon l'une des revendications précédentes,
**caractérisé en ce que** l'élément de couplage correspondant (15a ; 15b) délimite un canal de guidage (24a ; 24b) pour guider l'élément de couplage (14a ; 14b) dans l'échancrure (23a ; 23b) qui est au moins partiellement en forme de cercle.

4. Dispositif de poignée selon l'une des revendications précédentes,
**caractérisé en ce que** l'élément de couplage correspondant (15a ; 15b) forme un autre élément de centrage (17a ; 17b).

5. Dispositif de poignée selon l'une des revendications précédentes,
**caractérisé en ce que** l'élément de couplage (14c) et l'élément de couplage correspondant (15c) comprennent au moins une unité de blocage (57c) qui est destinée à bloquer les éléments de poignée (11c, 12c) dans au moins une position de blocage.

6. Dispositif de poignée selon l'une des revendications précédentes,
avec une unité de sécurisation (27a ; 27b ; 27c ; 27d) qui comprend un levier de fermeture (28a ; 28b ; 28c ; 28d) incluant un axe pivotant (29a ; 29b ; 29c ; 29d) pour le pivotement entre une position de relâche et une position de fixement pour l'unité-outil (25a ; 25b ; 25c ; 25d) et qui est conçue pour la retenue réversible de l'unité-outil (25a ; 25b ; 25c ; 25d) à au moins un des éléments de poignée (11a ; 11b ; 11c ; 11d ; 12a ; 12b ; 12c ; 12d),
**caractérisé en ce que** l'unité de sécurisation (27a) comprend une transmission à came (30a) avec deux surfaces inclinées (32a, 34a) glissant l'une sur l'autre, où avec un pivotement du levier de fermeture (28a) dans la position de relâche les surfaces inclinées (32a, 34a) glissent l'une de l'autre pour relâcher l'unité-outil (25a).

7. Dispositif de poignée selon la revendication 6,
**caractérisé en ce que** l'unité de sécurisation (27b) comprend un filetage (36b) et une vis à filetage (37b) qui - avec un pivotement du levier de fermeture (28b) - est vissée hors du filetage (36b). et relâche l'unité-outil (25b).

8. Dispositif de poignée selon la revendication 6 ou 7,
**caractérisé en ce que** l'unité-outil (25c) comprend un manche (26c) pour recevoir un outil aussi qu'une butée (42c) sur laquelle le manche (26c) est supporté
et que le levier de fermeture (28c) est conçu
pour le fixement directe de la butée (42c) sur l'un des éléments de poignée (11c, 12d) dans la position de fixement et
pour la relâche de la butée (42c) dans la position de relâche.

9. Dispositif de poignée selon l'une des revendications 6 à 8,
**caractérisé en ce que** l'unité-outil (25d) comprend au moins un chapiteau (63d, 64d) qui forme un outil avec le premier élément de poignée (11d) et qui est fixé sur le premier élément de poignée (11d) par le levier de fermeture (28d) dans la position de fixement.

10. Dispositif de poignée selon l'une des revendications 6 à 9,
**caractérisé en ce que** l'unité de sécurisation (27c ; 27d) comprend un autre levier de fermeture (53c ; 53d).

11. Système avec un dispositif de poignée (10a ; 10b ; 10c ; 10d) selon l'une des revendications précédentes et avec un outil, en particulier un outil chirurgical.
